# EUROPÄISCHE PATENTSCHRIFT

(11) **EP 3 234 593 B1**
(45) Veröffentlichungstag und Bekanntmachung des Hinweises auf die Patenterteilung: **04.02.2026**
(21) Anmeldenummer: 15819802.8
(22) Anmeldetag: 15.12.2015
(51) Int. Cl.: G01N 33/38, C04B 40/00, C04B 7/52

(54) **VORRICHTUNG SOWIE EIN VERFAHREN ZUR HERSTELLUNG UND ANALYSE EINER MEHRZAHL VON PROBEWERKSTOFFEN**
APPARATUS AND METHOD FOR PRODUCING AND ANALYZING A PLURALITY OF SAMPLE MATERIALS
DISPOSITIF ET PROCÉDÉ POUR LA FABRICATION ET L'ANALYSE D'UNE PLURALITÉ DE MATÉRIAUX D'ESSAI

(30) Priorität: 16.12.2014 DE 102014018489
(43) Veröffentlichungstag der Anmeldung: 25.10.2017
(73) Patentinhaber: thyssenkrupp Polysius GmbH, 59269 Beckum (DE); thyssenkrupp AG, 45143 Essen (DE)
(72) Erfinder: ENDERS, Michael, 48143 Münster (DE)
(74) Vertreter: thyssenkrupp Intellectual Property GmbH
(86) Internationale Anmeldenummer: PCT/EP2015/079880
(87) Internationale Veröffentlichungsnummer: WO 2016/096911

(56) Entgegenhaltungen:
- LIU C ET AL: "Effects of the granularity of raw materials on the hydration and hardening process of calcium phosphate cement", BIOMATERIALS, ELSEVIER, AMSTERDAM, NL, vol. 24, no. 23, 1 October 2003 (2003-10-01), pages 4103 - 4113, XP004436344, ISSN: 0142-9612, DOI: 10.1016/S0142-9612(03)00238-2
- CHEUNG J ET AL: "Impact of admixtures on the hydration kinetics of Portland cement", CEMENT AND CONCRETE RESEARCH, PERGAMON PRESS, ELMSFORD, NY, US, vol. 41, no. 12, 8 March 2011 (2011-03-08), pages 1289 - 1309, XP028310002, ISSN: 0008-8846, [retrieved on 20110310], DOI: 10.1016/J.CEMCONRES.2011.03.005
- IRASSAR E F ET AL: "Influence of limestone content, gypsum content and fineness on early age properties of Portland limestone cement produced by inter-grinding", CEMENT AND CONCRETE COMPOSITES, ELSEVIER APPLIED SCIENCE, BARKING, GB, vol. 33, no. 2, 1 February 2011 (2011-02-01), pages 192 - 200, XP027587153, ISSN: 0958-9465, [retrieved on 20110106]
- SHU-HUA LIU: "Hydration mechanism of low quality fly ash in cement-based materials", JOURNAL OF CENTRAL SOUTH UNIVERSITY, vol. 21, no. 11, 1 November 2014 (2014-11-01), Changsha, pages 4360 - 4367, XP093165448, ISSN: 2095-2899, Retrieved from the Internet <URL:https://link.springer.com/content/pdf/10.1007/s11771-014-2436-z.pdf> DOI: 10.1007/s11771-014-2436-z
- KUMAR S ET AL: "Mechanical activation of fly ash: Effect on reaction, structure and properties of resulting geopolymer", CERAMICS INTERNATIONAL, ELSEVIER, AMSTERDAM, NL, vol. 37, no. 2, 1 March 2011 (2011-03-01), pages 533 - 541, XP027558930, ISSN: 0272-8842, [retrieved on 20101028]
- LIU C ET AL: "Effects of the granularity of raw materials on the hydration and hardening process of calcium phosphate cement", BIOMATERIALS, ELSEVIER SCIENCE PUBLISHERS BV., BARKING, GB, vol. 24, no. 23, 1 October 2003 (2003-10-01), pages 4103 - 4113, XP004436344, ISSN: 0142-9612, DOI: 10.1016/S0142-9612(03)00238-2
- BOHNER M ET AL: "Combining particle size distribution and isothermal calorimetry data to determine the reaction kinetics of alpha-tricalcium phosphate-water mixtures", ACTA BIOMATERIALIA, ELSEVIER, AMSTERDAM, NL, vol. 2, no. 3, 1 May 2006 (2006-05-01), pages 343 - 348, XP028009588, ISSN: 1742-7061, [retrieved on 20060501], DOI: 10.1016/J.ACTBIO.2006.01.003
- E. FERNA'NDEZ* ET AL: "Production and characterization of new calcium phosphate bone cements in the CaHPO 4 a-Ca 3 (PO 4 ) 2 system: pH, workability and setting times", JOURNAL OF MATERIALS SCIENCE: MATERIALS IN MEDICINE, vol. 10, no. 4, 1 January 1999 (1999-01-01), United States, pages 223 - 230, XP055256369, ISSN: 0957-4530, DOI: 10.1023/A:1008958112257
- MEDINA C ET AL: "Rheological and calorimetric behaviour of cements blended with containing ceramic sanitary ware and construction/demolition waste", CONSTRUCTION AND BUILDING MATERIALS, ELSEVIER, NETHERLANDS, vol. 40, 28 December 2012 (2012-12-28), pages 822 - 831, XP028972565, ISSN: 0950-0618, DOI: 10.1016/J.CONBUILDMAT.2012.11.112

## Beschreibung

Die Erfindung betrifft eine Vorrichtung sowie ein Verfahren zur Herstellung und Analyse einer Mehrzahl von Probewerkstoffen, welche Proben eines hydraulischen Bindemittels sind.

### Stand der Technik

Anorganische Bindemittel werden in der Größenordnung von 4Gt jährlich in der Bauindustrie eingesetzt. Die Zusammensetzung der Bindemittel hat sich in den letzten Jahrzehnten verändert. An die Stelle traditioneller Zemente auf der Basis von Portlandzementklinker und Sulfatträger sind vielfach nachhaltige, kostengünstig herstellbare und hinsichtlich der Anwendungseigenschaften optimierte Kompositzemente aus Klinker, Zumahlstoffen und Sulfatträger getreten. Vor dem Hintergrund steigender Komplexität der Bindemittelzusammensetzung, der notwendigen Anpassung der Bindemittelfeinheit und der Anwendungseigenschaften sind der finanzielle Aufwand und der Zeitaufwand bei der Produktoptimierung und der Produktentwicklung angestiegen. Die Zielgrößen bei einer Produktoptimierung und der Produktentwicklung umfassen dabei zum Beispiel die Verarbeitbarkeit, das Erstarrungsverhalten und die Festigkeitsentwicklung. Abschließend muss die Leistungsfähigkeit des Bindemittels in der Hauptanwendung Beton untersucht werden.

Der hohe Materialbedarf für Betonuntersuchungen erfordert eine frühe Vorauswahl geeigneter Bindemittelzusammensetzungen und geeigneter Bindemittelfeinheitsbereiche. Die üblichen in der Baustoffindustrie angewandten physikalischen und in der EN 196 und EN 206 beschriebenen Analyseverfahren ermöglichen aufgrund des erheblichen Materialbedarfs lediglich eine Analyse einer geringen Probezahl und sind zudem zum Beispiel aufgrund der Prüfalter bis zu 28 Tagen sehr zeitaufwendig in ihrer Durchführung. Die Ermittlung der Auswirkung verschiedenster Parametervariationen auf die Reaktivität des Bindemittels ist daher sehr zeitaufwendig.

Aus KUMAR S ET AL: "Mechanical activation of fly ash: Effect on reaction, structure and properties of resulting geopolymer", CERAMICS INTERNATIONAL, ELSEVIER, AMSTERDAM, NL, Bd. 37, Nr. 2, 1. März 2011 (2011-03-01), Seiten 533-541, XP027558930, ISSN: 0272-8842 [gefunden am 2010-10-28] ist eine wissenschaftliche Arbeit zu Flugasche bekannt.

Aus LIU C ET AL: "Effects of the granularity of raw materials on the hydration and hardening process of calcium phosphate cement", BIOMATERIALS, ELSEVIER SCIENCE PUBLISHERS BV., BARKING, GB, Bd. 24, Nr. 23, 1. Oktober 2003 (2003-10-01), Seiten 4103-4113, XP004436344, ISSN: 0142-9612, DOI: 10.1016/S0142-9612(03)00238-2 ist eine wissenschaftliche Arbeit zur Untersuchung des Effekts der Partikelgröße auf die Hydratation und das Aushärten von Calciumphosphat-Zement bekannt.

Aus BOHNER M ET AL: "Combining particle size distribution and isothermal calorimetry data to determine the reaction kinetics of alpha-tricalcium phosphatewater mixtures", ACTA BIOMATERIALIA, ELSEVIER, AMSTERDAM, NL, Bd. 2, Nr. 3, 1. Mai 2006 (2006-05-01), Seiten 343-348, XP028009588, ISSN: 1742-7061, DOI: 10.1016/J.ACTBIO.2006.01.003 [gefunden am 2006-05-01] ist die Kombination von Partikelgrößenverteilung und Kalorimetrie zur Bestimmung der Reaktionskinetik von Calciumphosphat-Wasser-Mischungen bekannt.

Aus E. FERNA'NDEZ* ET AL: "Production and characterization of new calcium phosphate bone cements in the CaHPO 4 a-Ca 3 (PO 4) 2 system: pH, workability and setting times", JOURNAL OF MATERIALS SCIENCE: MATERIALS IN MEDICINE, Bd. 10, Nr. 4, 1. Januar 1999 (1999-01-01), Seiten 223-230, XP055256369, United States ISSN: 0957-4530, DOI: 10.1023/A:1008958112257 ist die Herstellung von künstlichem Knochenmaterial bekannt.

Aus MEDINA C ET AL: "Rheological and calorimetric behaviour of cements blended with containing ceramic sanitary ware and construction/demolition waste", CONSTRUCTION AND BUILDING MATERIALS, ELSEVIER, NETHERLANDS, Bd. 40, 28. Dezember 2012 (2012-12-28), Seiten 822-831, XP028972565, ISSN: 0950-0618, DOI: 10.1016/J.CONBUILDMAT.2012.11.112 ist eine wissenschaftliche Arbeit zum rheologischen und kalorimetrischen Verhalten von Zementmischungen bekannt.

Aus LIU C ET AL: "Effects of the granularity of raw materials on the hydration and hardening process of calcium phosphate cement", BIOMATERIALS, ELSEVIER, AMSTERDAM, NL, Bd. 24, Nr. 23, 1. Oktober 2003 (2003-10-01), Seiten 4103-4113, XP004436344, ISSN: 0142-9612, DOI: 10.1016/S0142-9612(03)00238-2 ist eine wissenschaftliche Arbeit zu Effekt der Granularität von Rohmaterialien bei der Hydratisierung und Härtung von Calciumphosphat-Zement bekannt.

Aus CHEUNG J ET AL: "Impact of admixtures on the hydration kinetics of Portland cement", CEMENT AND CONCRETE RESEARCH, PERGAMON PRESS, ELMSFORD, NY, US, Bd. 41, Nr. 12, 8. März 2011 (2011-03-08), Seiten 1289-1309, XP028310002, ISSN: 0008-8846, DOI: 10.1016/J.CEMCONRES.2011.03.005 [gefunden am 2011-03-10] ist eine wissenschaftliche Arbeit zum Einfluss von Zusatzstoffen auf die Hydratationskinetik von Portlandzement bekannt.

Aus IRASSAR E F ET AL: "Influence of limestone content, gypsum content and fineness on early age properties of Portland limestone cement produced by inter-grinding", CEMENT AND CONCRETE COMPOSITES, ELSEVIER APPLIED SCIENCE, BARKING, GB, Bd. 33, Nr. 2, 1. Februar 2011 (2011 -02-01), Seiten 192-200, XP027587153, ISSN: 0958-9465 ist eine wissenschaftliche Arbeit zur Untersuchung von Portland Zement Mischungen unterschiedlicher Zusammensetzungen und Mahlgraden bekannt.

### Offenbarung der Erfindung

Davon ausgehend ist es Aufgabe der vorliegenden Erfindung, eine Vorrichtung sowie ein Verfahren zur Herstellung und Analyse einer Mehrzahl von Probewerkstoffen, welche Proben eines hydraulischen Bindemittels darstellen, bereitzustellen, wobei auf einfache und schnelle Weise eine Analyse der Auswirkung einer Variation unterschiedlicher Parameter auf die Reaktivität des Bindemittels ermöglicht wird.

Diese Aufgabe wird erfindungsgemäß durch eine Vorrichtung mit den Merkmalen des unabhängigen Vorrichtungsanspruchs 1 sowie durch ein Verfahren mit den Merkmalen des unabhängigen Verfahrensanspruchs 9 gelöst. Vorteilhafte Weiterbildungen ergeben sich aus den abhängigen Ansprüchen.

Eine Vorrichtung zur Herstellung und Analyse einer Mehrzahl von Probewerkstoffen umfasst nach einem ersten Aspekt eine Mahleinrichtung zum Mahlen von Werkstoffkomponenten, zumindest eine erste Dosiereinrichtung zur Dosierung zumindest einer Werkstoffkomponente in die Mahleinrichtung, zumindest eine zweite Dosiereinrichtung zur Dosierung einer Anregerflüssigkeit zu der zumindest einen gemahlenen Werkstoffkomponente, eine Homogenisierungseinrichtung zur Homogenisierung der Werkstoffkomponenten und der Anregerflüssigkeit zu einem Probewerkstoff. Die Vorrichtung weist ferner eine Steuereinrichtung auf, die zumindest mit der Mahleinrichtung in Verbindung steht und derart ausgebildet ist, dass sie zumindest einen für die Mahlintensität der Mahleinrichtung charakteristischen Parameter variiert, sodass insbesondere die Korngröße der Werkstoffkomponenten verändert wird. Des Weiteren weist die Vorrichtung zumindest eine Messeinrichtung auf zum Ermitteln der Reaktivität des Probewerkstoffs.

Die Mahleinrichtung umfasst vorzugsweise eine Feinmühle, insbesondere eine Scheibenschwingmühle, die hinsichtlich der Umdrehungsgeschwindigkeit und der Mahldauer regelbar ist. Beispielsweise ist die Feinmühle automatisch betrieben und umfasst eine automatische Reinigungsvorrichtung. Die Mahleinrichtung umfasst ferner beispielsweise eine Vibrationsmühle, eine Kugelmühle, eine Vertikalrollenmühle oder eine Gutbettwalzenmühle.

Probenwerkstoffe sind hydraulische Bindemittel, die unterschiedliche Zusammensetzungen verschiedener Werkstoffkomponenten, wie beispielsweise Klinker, Sulfatträger oder Zumahlstoffe aufweisen. Zumahlstoffe sind beispielsweise Hüttensand, Flugasche, Puzzolan, Kalkstein oder kalzinierter Ton.

Eine Dosiereinrichtung zur Dosierung zumindest einer Werkstoffkomponente in die Mahleinrichtung umfasst vorzugsweise eine Wägezelle, die automatisiert, beispielsweise gravimetrisch oder volumetrisch, eine bestimmte Menge einer Werkstoffkomponente der Mahleinrichtung zuführt. Die Dosiereinrichtung steht direkt oder indirekt über ein Zwischenlager mit der Mahleinrichtung in Verbindung. Erfindungsgemäß ist für jede Werkstoffkomponente eine Dosiereinrichtung oder zumindest eine Dosiereinrichtung zur Dosierung einer Mehrzahl von Werkstoffkomponenten in die Mahleinrichtung angeordnet. Beispielsweise ist die Dosiereinrichtung in den Probenaufgabebereich der Mahleinrichtung integriert. Eine Dosiereinrichtung zur Dosierung einer Anregerflüssigkeit zu der zumindest einen gemahlenen Werkstoffkomponente umfasst beispielsweise eine regelbare Pumpe zum Beispiel in Form einer Peristaltik oder Pipette. Die Anregerflüssigkeit ist beispielsweise Wasser oder eine Alkalilösung.

Eine Homogenisierungseinrichtung zur Homogenisierung der Werkstoffkomponenten und der Anregerflüssigkeit zu einem Probewerkstoff umfasst vorzugsweise eine Vibrationseinrichtung oder eine Rühreinrichtung, wobei die Homogenisierung durch Vibration oder Rühren erfolgt.

Die Steuereinrichtung ist beispielsweise derart ausgebildet, dass sie die für die Mahlintensität charakteristischen Parameter elektrisch oder mechanisch variiert. Beispielsweise wird der gewünschte Parameterwert manuell an der Steuereinrichtung eingegeben oder von der Steuereinrichtung anhand weiterer Parameter, wie Dosiermenge oder Korngröße der Werkstoffkomponenten oder der Werkstoffzusammensetzung, ermittelt.

Ein für die Mahlintensität der Mahleinrichtung charakteristischer Parameter ist beispielsweise die Umdrehungsgeschwindigkeit, die Spaltbreite oder die Dauer der Mahlung der Werkstoffkomponenten. Die Mahlintensität beeinflusst die Korngröße der mit der Mahleinrichtung gemahlenen Werkstoffkomponenten, wobei eine hohe Mahlintensität eine geringe Korngröße und eine geringe Mahlintensität eine hohe Korngröße der gemahlenen Werkstoffkomponenten erzeugt. Durch eine Variation der Mahlintensität wird daher auch die Korngröße der Werkstoffkomponenten variiert.

Zur Herstellung eines Probewerkstoffs wird vorzugsweise über die Steuereinrichtung eine bestimmte Zusammensetzung des Probewerkstoffs oder mehrerer Probenwerkstoffe eingestellt, wobei die Anteile bestimmter Werkstoffkomponenten beispielsweise manuell an der Steuereinrichtung einstellbar oder durch die Steuereinrichtung ermittelbar sind. Die Dosiereinrichtung dosiert eine vorbestimmte Menge an Werkstoffkomponenten in die Mahleinrichtung, in der die Werkstoffkomponenten beispielsweise einzeln oder zusammen vermahlen werden. Vorzugsweise wird der gemahlene Probewerkstoff in einen bevorzugt flüssigkeitsundurchlässigen Probebehälter gefüllt, der anschließend der Homogenisierungseinrichtung zugeführt wird. Im Anschluss an die Dosierung der Anregerflüssigkeit zu den gemahlenen Werkstoffkomponenten in dem Probenbehälter wird der homogenisierte Probewerkstoff der Messeinrichtung zur Ermittlung der Reaktivität zugeführt.

Eine Steuereinrichtung, die derart ausgebildet ist, dass sie zumindest einen für die Mahlintensität der Mahleinrichtung charakteristischen Parameter variiert, ermöglicht die Herstellung einer Vielzahl von Probewerkstoffen deren Korngröße in bekannter Weise variiert. Auf einfache Weise ist eine große Anzahl von Probewerkstoffen unterschiedlicher Feinheit herstellbar und in der Messeinrichtung analysierbar. Dies ermöglicht eine Ermittlung der Reaktivität der Probewerkstoffe in Abhängigkeit der Korngröße des Probewerkstoffs auf einfache Weise, wobei eine hohe Datendichte, bezogen auf die Variation der Korngröße, erzielt wird. Eine genaue Analyse, insbesondere nichtlinearer Zusammenhänge zwischen der Reaktivität und der Korngröße des Probewerkstoffs, sind analysierbar, sodass eine zuverlässige Optimierung dieser Parameter ermöglicht wird.

Gemäß einer ersten Ausführungsform ist die Messeinrichtung eine kalorimetrische Messeinrichtung, insbesondere ein isothermales Wärmeflusskaloriemeter. Eine kalorimetrische Messeinrichtung ermittelt die von dem Probewerkstoff abgegebene Reaktionswärme. Die freigegebene Reaktionswärme und der Verlauf der Wärmeabgabe über die Zeit sind charakteristisch für die Reaktivität eines Probewerkstoffs, insbesondere eines Bindemittels. Die kalorimetrische Messeinrichtung ermöglicht eine einfache und schnelle Ermittlung der Reaktivität des Probewerkstoffs.

Die Steuereinrichtung steht erfindungsgemäß mit der Dosiereinrichtung in Verbindung und ist derart ausgebildet, dass sie die Dosiermenge der Werkstoffkomponente steuert. Dies ermöglicht eine Variation der Menge der Werkstoffkomponenten und der Korngröße des Probewerkstoffs mittels der Steuereinrichtung, wobei eine Vielzahl von Probewerkstoffen mit in bekannter Weise variierten Parametern, wie Korngröße und Zusammensetzung des Probewerkstoffs, herstellbar sind und somit eine Analyse der Reaktivität in Abhängigkeit der Zusammensetzung des Probewerkstoffs ermöglicht wird.

Zumindest eine Dosiereinrichtung ist gemäß einer weiteren Ausführungsform beheizbar. Dies ermöglicht eine Dosierung eines Sulfatträgers bei gleichzeitiger thermischer Entwässerung und so die gezielte Einstellung und Nutzung teilweiser oder vollständiger Sulfatträger in den Probewerkstoffen.

Die Vorrichtung weist ferner eine Brecheinrichtung auf, die der Mahleinrichtung vorgeschaltet ist. Die Brecheinrichtung ermöglicht eine Vorzerkleinerung von Werkstoffkomponenten, die beispielsweise Granalien mit einer Größe von etwa 3-10 mm umfassen. Eine Brecheinrichtung erhöht ferner die Dosiergenauigkeit von Werkstoffkomponenten mit einer großen Korngröße.

Gemäß einer weiteren Ausführungsform weist die Vorrichtung eine rheometrische Messeinrichtung zur Ermittlung der rheologischen Eigenschaften des Probewerkstoffs auf. Rheometrische Messverfahren ermöglichen eine einfache Ermittlung der Abhängigkeit der rheometrischen Eigenschaften des Probewerkstoffs von Parametern, wie Korngröße und Zusammensetzung des Probewerkstoffs.

Die Vorrichtung weist nach einer weiteren Ausführungsform ein Probenlager mit einer Mehrzahl von Probebehältern zur Aufnahme eines Probewerkstoffs auf. Ein solches Probenlager ermöglicht die gleichzeitige Analyse einer Mehrzahl von Probebehältern mit Probewerkstoffen in einer Messeinrichtung, wie einer kalorimetrischen oder einer rheologischen Messeinrichtung. Dies verkürzt die Zeit zur Analyse einer Mehrzahl von Probewerkstoffen erheblich.

Die Messeinrichtung ist gemäß einer weiteren Ausführungsform derart ausgebildet, dass sie die Reaktivität einer Mehrzahl Probewerkstoffen gleichzeitig ermittelt.

Die Vorrichtung umfasst gemäß einer weiteren Ausführungsform ferner eine der Mahleinrichtung nachgeschaltete Dosiereinrichtung zum Dosieren zumindest eines Zementadditivs zu der zumindest einen gemahlenen Werkstoffkomponente. Vorzugsweise steht die Steuereinrichtung mit der Dosiereinrichtung in Verbindung, sodass die Menge an Zementadditiven über die Steuereinrichtung einstellbar ist. Bei Zementadditiven handelt es sich beispielsweise um Mahlhilfen wie das DEG (Di-ethylenglykol) oder TEA (Triethanolamin) oder Zementeigenschaften modifizierende organische oder anorganische Zusatzmittel (z.B. TIPA, CaCl2).

Die Steuereinrichtung steht vorzugsweise mit der Dosiereinrichtung zur Dosierung der Anregerflüssigkeit in Verbindung, sodass die Menge an Anregerflüssigkeit über die Steuereinrichtung steuerbar ist und die Reaktivität des Probewerkstoffs in Abhängigkeit der Menge der Anregerflüssigkeit ermittelbar ist. Die Dosierung erfolgt z.B. über eine Peristaltik oder eine Pipette.

Die Vorrichtung ist vorzugsweise in einer temperaturgeregelten isothermen Umgebung, wie beispielsweise einem Laborraum, angeordnet. Dies ermöglicht beispielsweise, wenn die Messeinrichtung ein isothermales Wärmeflusskalorimeter umfasst, eine Entlastung des Thermostaten der Messeinrichtung, wobei die zur optimalen Messung benötigte Temperatur in der gesamten Vorrichtung eingestellt wird.

Die beanspruchte Erfindung beinhaltet ein Verfahren zur Herstellung und Analyse einer Mehrzahl von Probewerkstoffen welche Proben eines hydraulischen Bindemittels sind, entsprechend dem Anspruch 9.

Die mit Bezug auf die Vorrichtung voran beschriebenen Vorteile treffen in verfahrensmäßiger Entsprechung auf das Verfahren zur Herstellung und Analyse einer Mehrzahl von Probewerkstoffen zu.

Gemäß einer ersten Ausführungsform wird bei dem Schritt b) die Drehzahl der Mahleinrichtung und/ oder die Mahldauer der zumindest einen Werkstoffkomponente variiert.

Ferner werden gemäß einer weiteren Ausführungsform die rheologischen Eigenschaften der Mehrzahl von Probewerkstoffen ermittelt.

Die zumindest eine Werkstoffkomponente wird nach einer weiteren Ausführungsform vor dem Schritt b) gebrochen.

Der Schritt a) umfasst nach einer weiteren Ausführungsform das Erwärmen zumindest einer Werkstoffkomponente.

Vor dem Schritt d) wird nach einer weiteren Ausführungsform die Korngröße zumindest eines Teils der gemahlenen Werkstoffkomponenten ermittelt. Die Bestimmung der Feinheit oder Kornverteilung der Probe, mit zum Beispiel Sieben oder einem Lasergranulometer, ermöglicht die Korrelation der Messergebnisse mit einem Korngrößenparameter und die leichte Umsetzung des neuentwickelten Werkstoffs in die industrielle Produktion.

### Bevorzugte Ausführungsbeispiele der Erfindung

Die Erfindung ist nachfolgend anhand mehrerer Ausführungsbeispiele mit Bezug auf die beiliegenden Figuren näher erläutert.
- Fig. 1: zeigt eine schematische Darstellung einer Vorrichtung zur Analyse eines Probewerkstoffs gemäß einem Ausführungsbeispiel.
- Fig. 2: zeigt eine schematische Darstellung einer Vorrichtung zur Analyse eines Probewerkstoffs gemäß einem weiteren Ausführungsbeispiel.

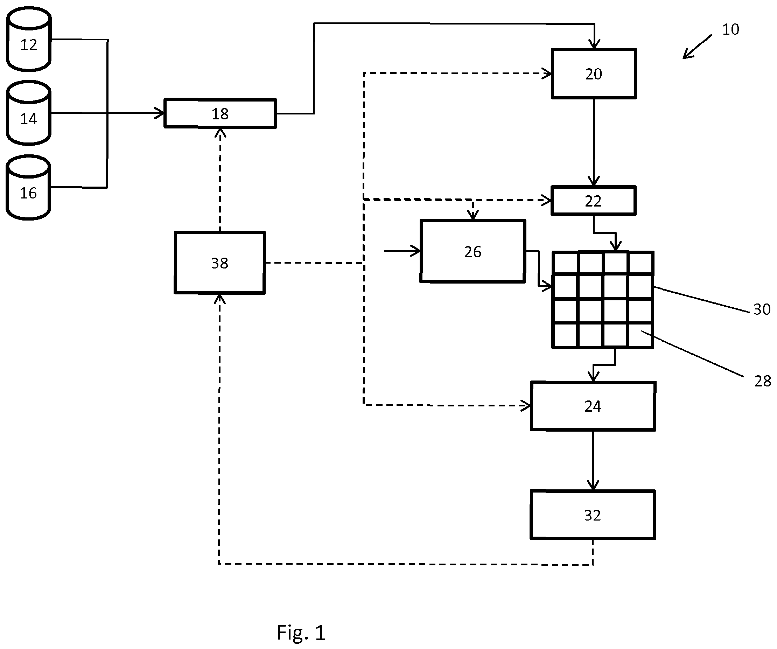

Fig. 1 zeigt eine schematische Darstellung einer Vorrichtung 10 zur Analyse eines Probewerkstoffs. Die Analysevorrichtung 10 weist beispielshaft drei Behälter 12, 14 und 16 auf, in denen jeweils eine Werkstoffkomponente gelagert ist. Die Darstellung von drei Behältern 12, 14, 16 ist nur beispielhaft, es ist denkbar, eine Vielzahl von Behältern mit jeweils einer Werkstoffkomponente in der Analysevorrichtung 10 vorzusehen.

Bei den Werkstoffkomponenten handelt es sich insbesondere um Werkstoffkomponenten eines Bindemittels, wie Zement. Beispielsweise werden Klinker, wie Portlandzementklinker oder Sulfoaluminatklinker, mit einem Sulfatträger und Zumahlstoffen zur Herstellung von Bindemitteln verwendet. Bei einem Sulfatträger handelt es sich beispielsweise um Gips, Halbhydrat, Anhydrit oder andere Stoffe mit erhöhtem SO₃ Gehalt. Zumahlstoffe sind beispielsweise latent hydraulische Materialien, Hüttensand, Braunkohlenflugasche, Silikastaub, oder Puzzolane, wie Steinkohlenflugasche, natürliche Puzzolane, synthetische Puzzolane, oder Füllermaterialien, wie Kalkstein. Die Werkstoffkomponenten sind in den Behältern 12, 14, 16 vorzugsweise als Brechgut mit einer Korngröße von bis zu 3mm, als Pulver mit einer Korngröße von bis zu etwa 0,09mm oder als Granalien mit einer Korngröße von etwa 3 bis 10mm gelagert.

Die Analysevorrichtung 10 weist ferner eine erste Dosiereinrichtung 18 auf, die mit den Behältern 12, 14 und 16 in Verbindung steht. Die Dosiereinrichtung 18 ist beispielsweise eine Wägezelle, eine Waage oder eine volumetrische Messeinrichtung. Die Dosiereinrichtung führt eine bestimmte Menge der Werkstoffkomponenten aus dem jeweiligen Behälter 12, 14, 16 ab. Die Dosiergenauigkeit der Dosiereinrichtung ist bezogen auf die Gesamteinwaage etwa 0.01 - 1 %, vorzugsweise 0,007% - 0,25% höchstvorzugshalber etwa 0.5 %.

Der Dosiereinrichtung 18 ist eine Mahleinrichtung 20 nachgeschaltet. Bei der Mahleinrichtung 20 handelt es sich beispielsweise um eine Scheibenschwingmühle oder eine Vibrationsmühle. Die Dosiereinrichtung 18 dosiert eine bestimmte Menge einer Werkstoffkomponente aus einem der Behälter 12, 14 und 16 in die Mahleinrichtung 20. Die Werkstoffkomponenten werden beispielweise direkt von einem der Behälter 12, 14, 16 über die Dosiereinrichtung 18 der Mahleinrichtung 20 zugeführt oder in einem nicht darstellten Zwischenlager zusammengeführt und anschließend zusammen der Mahleinrichtung 20 zugeführt.

An die Mahleinrichtung 20 schließt sich eine weitere optionale Dosiervorrichtung 22 sowie eine Homogenisierungseinrichtung 24, eine zweite Dosiereinrichtung 26 zur Dosierung einer Anregerflüssigkeit und ein Probenlager 30 mit einer Mehrzahl von Probebehältern 28 an.

Die Analysevorrichtung 10 weist ferner eine kalorimetrische Messeinrichtung 32 zur Ermittlung der Reaktivität eines Probewerkstoffs sowie eine rheometrische Messeinrichtung 34 zur Ermittlung der Verarbeitungseigenschaften des Probewerkstoffs auf.

Des Weiteren weist die Analysevorrichtung 10 eine Steuerungseinrichtung 38 auf, die mit der ersten Dosiereinrichtung 18, der Mahleinrichtung 20, der Anregerflüssigkeits- Dosiereinrichtung 26, der Homogenisiereinrichtung 24 sowie den Messeinrichtungen 32 und 34 in Verbindung steht.

In einem Analyseverfahren mittels der Analyseeinrichtung 10 ist über die Steuereinrichtung 38 eine bestimmte Zusammensetzung des Probewerkstoffs einstellbar. Die Anteile der Werkstoffkomponenten aus den Behältern 12, 14, 16 an dem Probewerkstoff sind beispielsweise manuell an der Steuereinrichtung 38 einstellbar oder durch die Steuereinrichtung 38 bei einem vorgegebenen Intervall berechenbar, sodass eine Mehrzahl von Probewerkstoffen mit einer über einen bestimmten Mengenintervall variierenden Anteil einer Werkstoffkomponente analysierbar ist. Die Steuerungseinrichtung 38 steht mit der Dosiereinrichtung 18 derart in Verbindung, dass die an der Steuereinrichtung 38 eingestellte Dosiermenge an Werkstoffkomponenten über die Dosiereinrichtung 18 aus den Behältern 12, 14, 16 zur Mahleinrichtung dosiert wird. Die Werkstoffkomponenten werden anschließend einzeln oder gemeinsam in der Mahleinrichtung 20 gemahlen. Bei einer gemeinsamen Vermahlung der Werkstoffkomponenten werden die Werkstoffkomponenten nacheinander in einem in Fig. 1 nicht dargestellten Probebehälter dosiert und anschließend der Mahleinrichtung zugeführt.

Über die Steuereinrichtung 38 ist beispielsweise die Umdrehungsgeschwindigkeit oder die Vibrationsgeschwindigkeit der Mahleinrichtung 20 sowie die Dauer des Mahlvorgangs und somit die Mahlintensität einstellbar. Eine bestimmte Feinheit des Probewerkstoffs ist somit über die Steuereinrichtung 38 an der Mahleinrichtung 20 einstellbar.

Im Anschluss an die Mahleinrichtung 20 wird der Probewerkstoff über eine Dosiervorrichtung 22 in einen Probebehälter 28 dosiert. Die Dosiervorrichtung 22 ermittelt beispielsweise das in den Probebehälter 28 eingefüllte Nettogewicht des Probewerkstoffs. Beispielsweise ermittelt die Dosiereinrichtung das Nettogewicht des Probebehälters 28, das Gesamtgewicht des Probehälters 28 mit dem Probewerkstoff und/ oder das Nettogewicht des in den Probebehälter 28 eingefüllten Probewerkstoffs.

Der Probebehälter ist in einem Probenlager 30 angeordnet, das eine Mehrzahl von Probebehältern 28 aufweist, die beispielsweise jeweils unterschiedliche Probewerkstoffe beinhalten. In dem In Fig. 1 dargestellten Ausführungsbeispiel umfasst das Probenlager sechzehn Probebehälter 28, die in vier Reihen angeordnet sind.

Die voran beschriebenen Schritte werden vorzugsweise unter Variation der von der Steuereinrichtung 38 steuerbaren Parameter wiederholt, sodass eine Mehrzahl unterschiedlicher Probenzusammensetzungen in unterschiedlichen Probebehältern 28 in dem Probenlager 30 angeordnet sind.

Über die Anregerflüssigkeits-Dosiereinrichtung 26 wird dem Probebehälter 28 eine Anregerflüssigkeit zugeführt. Ist eine der Werkstoffkomponenten Portlandzementklinker oder Sulfoaluminatklinker, wird als Anregerflüssigkeit Wasser in den Probebehälter 28 dosiert. Ist eine der Werkstoffkomponenten ein Geopolymer, wird als Anregerflüssigkeit eine Alkalilösung in den Probebehälter 28 dosiert. Die Anregerflüssigkeits-Dosiereinrichtung 26 weist beispielsweise eine Heizvorrichtung zum Erwärmen der Anregerflüssigkeit, insbesondere auf die Temperatur der kalorimetrischen Messeinrichtung 32 auf. Die Dosiergenauigkeit der Dosiereinrichtung 26 umfasst maximal etwa 0,001 bis 1 %, vorzugsweise 0,007 - 0,25%, höchstvorzugshalber etwa 0,5% der angestrebten Dosiermenge.

Insbesondere wird über die Anregerflüssigkeits-Dosiereinrichtung 26 eine Anregerflüssigkeit zu den Werkstoffkomponenten in dem Probebehälter 28 dosiert, sodass das Verhältnis von Flüssigkeit zu Probewerkstoff etwa 0,25 - 1,2, insbesondere 0,4 - 0,6, besonders bevorzugt 0,45 - 0,55 beträgt.

In der Homogenisierungseinrichtung 24 werden die Anregerflüssigkeit und die Werkstoffkomponenten in dem Probebehälter zu einem Probewerkstoff homogenisiert. Die Homogenisierungseinrichtung 24 umfasst beispielweise eine Vibrationsvorrichtung, durch welche der Probebehälter mit einer Vibration beaufschlagt wird. Die Homogenisierung 24 kann ferner durch mechanisches Mischen, wie Rühren oder Schlagen durchgeführt werden.

Der Probebehälter 28 in dem Probenlager 30 wird anschließend der kalorimetrischen Messeinrichtung 32 zugeführt. Es ist ebenfalls denkbar, den Probebehälter einzeln, ohne Probenlager 30, der kaloriemetrischen Messeinrichtung 32 zuzuführen.

Die Zugabe der Anregerflüssigkeit zu den Werkstoffomponenten startet den Hydratationsprozess, wobei die in den Werkstoffkomponenten gespeicherte Energie in Form von Reaktionswärme freigesetzt wird. Der Probebehälter 28 wird daher unmittelbar nach der Zugabe der Anregerflüssigkeit und der anschließenden Homogenisierung der Messeinrichtung 32 zugeführt, sodass vorzugsweise die gesamte freigesetzte Reaktionswärme messbar ist. Beispielsweise handelt es sich bei der Reaktionswärme um zuvor durch die Abkühlung in einem Klinkerkühler in den Mineralphasen eingespeicherte Energie im Klinker. Diese Reaktionswärme ist charakteristisch für die Reaktivität des Gemisches der Werkstoffkomponenten. Die bei der Hydratation kumulativ freigesetzte Energie zu einem Zeitpunkt entspricht dem gewichteten Mittel des Beitrags der einzelnen Werkstoffkomponenten. Der Hydratationsgrad und die Hydratationsgeschwindigkeit wird über die mineralogische Zusammensetzung der Werkstoffkomponenten, insbesondere dem Klinker, den zugesetzten Sulfatträger und die bei der Aufmahlung der Werkstoffkomponenten erzeugte Oberfläche bestimmt. Die Hydratationsreaktion verläuft isochemisch, wobei die neu gebildeten Hydratphasen zu wesentlichen Anteilen röntgenamorph sind.

Die kalorimetrische Messeinrichtung 32 ist derart ausgebildet, dass sie die von dem Probewerkstoff abgegebene Wärme über einen bestimmten Zeitraum ermittelt.

Kommerziell verfügbare Kalorimeter, wie beispielsweise isothermale Wärmeflusskalorimeter, ermöglichen die parallele Bestimmung der Hydratationswärme mehrerer unterschiedlicher Probewerkstoffe über eine bestimmte Zeit. Bekannte Kalorimeter weisen mehrere Messkanäle auf, in denen gleichzeitig unterschiedliche Probewerkstoffe analysiert werden. Die kalorimetrische Messeinrichtung 32 ist derart ausgebildet, dass geringe Probenmengen, wie beispielsweise 1- 10 g, vorzugsweise 3 - 8g, höchstvorzugshalber etwa 5g, zur Ermittlung der Reaktivität des Probewerkstoffs ausreichen. Die Messeinrichtung 32 ermittelt die Hydratationswärme beispielsweise über einen Messzeitraum von etwa 1 - 8, insbesondere 4 -6, vorzugsweise 5 Tagen bei einer Temperatur im Kalorimeter von etwa 20 - 45 °C, insbesondere 20 - 27°C. Durch Anhebung der Temperatur auf etwa 45 °C, sind auch geringe Messzeit unter einem Tag denkbar.

Die kalorimetrische Messeinrichtung 32 übermittelt das Messergebnis an die Steuereinrichtung 38. Die Steuereinrichtung 38 ist ferner derart ausgebildet, dass sie mittels der durch die kalorimetrische Messeinrichtung ermittelten von dem Probewerkstoff abgegebene Wärme eine Reaktivität des Probewerkstoffs ermittelt.

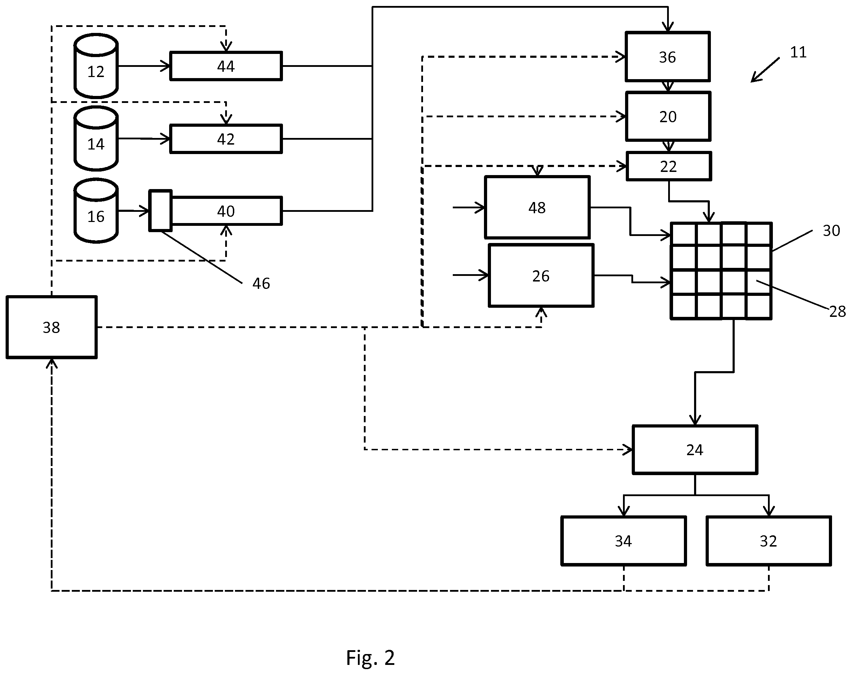

Zwischen den Elementen der Analysevorrichtung 10 sind beispielsweise Transportvorrichtungen, wie Förderbänder, mobile Roboter oder ortsfeste Roboter mit großer Reichweite, angeordnet, die in Fig. 1 und 2 schematisch als Pfeile dargestellt sind.

Fig. 2 zeigt eine schematische Darstellung einer Vorrichtung 11 zur Analyse eines Probewerkstoffs gemäß einem weiteren Ausführungsbeispiel. Die Analysevorrichtung 11 weist alle Elemente der mit Bezug auf Fig. 1 beschriebenen Analysevorrichtung 10 auf, wobei die Dosiereinrichtung 18 der Analyseeinrichtung 10 durch drei Dosiereinrichtungen 40, 42, 44 ersetzt wurde, wobei die Dosiereinrichtung 40 zusätzlich eine Heizeinrichtung 46 aufweist. Weiterhin weist die Analyseeinrichtung 11 der Fig. 2 eine weitere der Mahleinrichtung 20 vorgeschaltete Brecheinrichtung 36 sowie eine dritte weitere Dosiereinrichtung 48 zur Dosierung von festen oder flüssigen Zusatzstoffen, wie beispielsweise Mahlhilfsmittel (z.B. DEG, TEA) oder Zementadditive (z.B. TIPA, CaCl2) in den Probebehälter 28 auf.

Die Analysevorrichtung 11 weist ferner eine rheometrische Messeinrichtung 34 auf, zur Ermittlung der rheologischen Eigenschaften der Probewerkstoffe in den Probebehältern 28 des Probenlagers 30.

Das mit der Analysevorrichtung 11 durchführbare Analyseverfahren entspricht im Wesentlichen dem mit Bezug auf Fig. 1 beschriebenen Analyseverfahren, wobei jeder der Behälter 12, 14, 16 eine Dosiereinrichtung 40, 42, 44 zugeordnet ist, die eine mittels der Steuereinrichtung 38 einstellbare Menge an Werkstoffkomponenten an die Brecheinrichtung 36 dosiert. Die Dosiereinrichtung 40, die dem Behälter 16 zugeordnet ist, weist eine Heizeinrichtung 46 auf, durch welche die Werkstoffkomponente aus dem Behälter 16 heizbar ist. Bei der Werkstoffkomponente in dem Behälter 16 handelt es sich beispielsweise um einen Sulfatträger, der mittels der Heizeinrichtung 46 erhitzt und somit thermisch entwässert wird.

Die Brecheinrichtung 36 bricht insbesondere Werkstoffkomponenten großer Korngröße, wie Granalien mit einer Korngröße von etwa 3 bis 10mm, vor der Zufuhr der Werkstoffkomponenten an die Mahleinrichtung 20.

Ferner weist das Analyseverfahren mittels der Analysevorrichtung 11 eine rheometrische Messung mittels der rheometrischen Messeinrichtung 34 auf. Die rheometrische Messeinrichtung 34 ermittelt von zumindest einem Teil des Probewerkstoffs in den Probebehältern 28 des Probenlagers 30 die rheologischen Eigenschaften, wie beispielsweise die Fließgrenze.

Bei den Analysevorrichtungen 10 und 11 sind über die Steuereinrichtung Parameter, wie die Massenanteile der Werkstoffkomponenten aus den Behältern 12, 14, 16 in dem Probewerkstoff, der Mahlungsgrad der Werkstoffkomponenten und die Menge an Anregerflüssigkeit und Zusatzstoffe des Probewerkstoffs einstellbar, sodass eine Vielzahl von Probewerkstoffe herstellbar sind, bei welchen ein oder mehrere Parameter in bekannter Weise variieren. Eine Analyse der Mehrzahl von Probewerkstoffen eines Probenlager 30 gleichzeitig in der kalorimetrischen Messeinrichtung 32 ermöglicht eine Ermittlung der Reaktivität eines Werkstoffs in Abhängigkeit bestimmter Parameter, wie Korngröße und Zusammensetzung des Werkstoffs auf einfache Weise. Ferner ist es mit der Analyseinrichtung 10, 11 möglich, auf einfache Weise eine große Anzahl an Probewerkstoffen herzustellen und somit eine hohe Datendichte, bezogen auf die Variation der Parameter, zu erzeugen. Dies ermöglicht eine genaue Analyse, insbesondere nichtlinearer Zusammenhänge zwischen der Reaktivität oder den rheologischen Eigenschaften und den Parametern der Probewerkstoffe, wie Zusammensetzung und Korngröße.

### Bezugszeichenliste

- 10: Analysevorrichtung
- 11: Analysevorrichtung
- 12: Speicher
- 14: Speicher
- 16: Speicher
- 18: erste Dosiereinrichtung
- 20: Mahleinrichtung
- 22: Dosiervorrichtung
- 24: Homogenisiereinrichtung
- 26: zweite Dosiereinrichtung zur Dosierung einer Anregerflüssigkeit
- 28: Probebehälter
- 30: Probenlager
- 32: Messeinrichtung
- 34: Messeinrichtung
- 36: Brecheinrichtung
- 38: Steuerungseinrichtung
- 40: Dosiereinrichtung
- 42: Dosiereinrichtung
- 44: Dosiereinrichtung
- 46: Heizeinrichtung
- 48: dritte Dosiereinrichtung

## Patentansprüche

1. Vorrichtung (10, 11) zur Herstellung und Analyse einer Mehrzahl von Probewerkstoffen, wobei die Probewerkstoffe hydraulische Bindemittel sind, und wobei die Probenwerkstoffe unterschiedliche Zusammensetzungen verschiedener Werkstoffkomponenten aufweisen, umfassend
eine Mahleinrichtung (20) zum Mahlen von Werkstoffkomponenten,
zumindest eine erste Dosiereinrichtung (18; 40, 42, 44) zur Dosierung, einer Mehrzahl von Werkstoffkomponenten in die Mahleinrichtung (20),
zumindest eine zweite Dosiereinrichtung (26) zur Dosierung einer Anregerflüssigkeit zu der zumindest einen gemahlenen Werkstoffkomponente,
eine Homogenisierungseinrichtung (24) zur Homogenisierung der Werkstoffkomponenten und der Anregerflüssigkeit zu einem Probewerkstoff,
**dadurch gekennzeichnet, dass** die Vorrichtung eine Steuereinrichtung (38) aufweist, die zumindest mit der Mahleinrichtung (20) in Verbindung steht und derart ausgebildet ist, dass sie zumindest einen für die Mahlintensität der Mahleinrichtung (20) charakteristischen Parameter variiert, sodass insbesondere die Korngröße der Werkstoffkomponenten verändert wird, wobei die Steuereinrichtung (38) mit der zumindest einen ersten Dosiereinrichtung (18; 40, 42, 44) in Verbindung steht und derart ausgebildet ist, dass sie die Dosiermenge der Werkstoffkomponenten steuert und
zumindest eine Messeinrichtung (32) zum Ermitteln der Reaktivität des Probewerkstoffs angeordnet ist.

2. Vorrichtung (10; 11) nach Anspruch 1, wobei die Messeinrichtung (32) eine kalorimetrische Messeinrichtung ist.

3. Vorrichtung (10; 11) nach einem der vorangehenden Ansprüche, wobei die zumindest eine erste Dosiereinrichtungen (18; 40, 42, 44) beheizbar ist.

4. Vorrichtung (10; 11) nach einem der vorangehenden Ansprüche, wobei die Vorrichtung (10; 11) eine Brecheinrichtung (36) aufweist, die der Mahleinrichtung (20) vorgeschaltet ist.

5. Vorrichtung (10; 11) nach einem der vorangehenden Ansprüche, wobei die Vorrichtung (10; 11) eine rheometrische Messeinrichtung (34) zur Ermittlung der rheologischen Eigenschaften des Probewerkstoffs aufweist.

6. Vorrichtung (10; 11) nach einem der vorangehenden Ansprüche, wobei die Vorrichtung (10; 11) ein Probenlager (30) mit einer Mehrzahl von Probebehältern (28) zur Aufnahme eines Probewerkstoffs aufweist.

7. Vorrichtung (10; 11) nach einem der vorangehenden Ansprüche, wobei die Messeinrichtung (32) derart ausgebildet ist, dass sie die Reaktivität einer Mehrzahl Probewerkstoffen gleichzeitig ermittelt.

8. Vorrichtung (10; 11) nach einem der vorangehenden Ansprüche, umfassend zumindest eine der Mahleinrichtung (20) nachgeschaltete dritte Dosiereinrichtung (48) zum Dosieren zumindest eines Zementadditivs zu der zumindest einen gemahlenen Werkstoffkomponente.

9. Verfahren zur Herstellung und Analyse einer Mehrzahl von Probewerkstoffen welche Proben eines hydraulischen Bindemittels sind, wobei die hydraulischen Bindemittel unterschiedliche Zusammensetzungen verschiedener Werkstoffkomponenten aufweisen, aufweisend die Schritte:
a) Dosieren einer Mehrzahl von Werkstoffkomponenten in eine Mahleinrichtung (20)
b) Mahlen der zumindest einen Werkstoffkomponente in der Mahleinrichtung (20)
c) Dosieren einer Anregerflüssigkeit zu der zumindest einen Werkstoffkomponente
d) Homogenisieren der zumindest einen Werkstoffkomponente und der Anregerflüssigkeit zu einem Probewerkstoff
**dadurch gekennzeichnet, dass** die Schritte a) bis d) zur Herstellung einer Mehrzahl von Probewerkstoffen wiederholt werden, wobei zumindest ein für die Mahlintensität charakteristischer Parameter an der Mahlanlage (20) variiert wird,
wobei das Verfahren des Weiteren den Schritt des Ermittelns der Reaktivität der Mehrzahl von Probewerkstoffen mittels einer kalorimetrischen Messeinrichtung (32) aufweist.

10. Verfahren nach Anspruch 9, wobei bei dem Schritt b) die Drehzahl der Mahleinrichtung und/ oder die Mahldauer der zumindest einen Werkstoffkomponente variiert wird.

11. Verfahren nach einem der Ansprüche 9 oder 10, wobei die rheologischen Eigenschaften der Mehrzahl von Probewerkstoffen ermittelt werden.

12. Verfahren nach einem der Ansprüche 9 bis 11, wobei die zumindest eine Werkstoffkomponente vor dem Schritt b) gebrochen wird.

13. Verfahren nach einem der Ansprüche 9 bis 12, wobei der Schritt a) das Erwärmen zumindest einer Werkstoffkomponente umfasst.

14. Verfahren nach einem der Ansprüche 9 bis 13, wobei vor dem Schritt d) die Korngröße zumindest eines Teils der gemahlenen Werkstoffkomponenten ermittelt wird.

## Claims

1. An apparatus (10, 11) for producing and analyzing a plurality of sample materials, wherein the sample materials are hydraulic binder and wherein the sample materials contain different compositions of various material components, which comprises
a milling device (20) for milling material components,
at least one first metering device (18; 40, 42, 44) for metering at least one material component into the milling device (20),
at least one second metering device (26) for metering an activator liquid into the at least one milled material component,
a homogenization device (24) for homogenizing the material components and the activator liquid to give a sample material,
**characterized in that** the apparatus has a control device (38) which is connected at least to the milling device (20) and is configured in such a way that it varies at least one parameter characteristic for the milling intensity of the milling device (20) so that, in particular, the particle size of the material components is altered, wherein the control device (38) is connected to the at least one first metering device (18; 40, 42, 44) and is configured in such a way that it controls the metered amount of the material component and
at least one measuring device (32) for determining the reactivity of the sample material is provided.

2. The apparatus (10; 11) as claimed in claim 1, wherein the measuring device (32) is a calorimetric measuring device.

3. The apparatus (10; 11) as claimed in any of the preceding claims, wherein the at least one first metering device (18; 40, 42, 44) is heatable.

4. The apparatus (10; 11) as claimed in any of the preceding claims, wherein the apparatus (10; 11) has a crushing device (36) which is located upstream of the milling device (20).

5. The apparatus (10; 11) as claimed in any of the preceding claims, wherein the apparatus (10; 11) has a rheometric measuring device (34) for determining the rheological properties of the sample material.

6. The apparatus (10; 11) as claimed in any of the preceding claims, wherein the apparatus (10; 11) has a sample store (30) having a plurality of sample containers (28) for accommodating a sample material.

7. The apparatus (10; 11) as claimed in any of the preceding claims, wherein the measuring device (32) is configured in such a way that it simultaneously determines the reactivity of a plurality of sample materials.

8. The apparatus (10; 11) as claimed in any of the preceding claims, comprising at least one third metering device (48) for metering at least one cement additive into the at least one milled material component located downstream of the milling device (20).

9. A process for producing and analyzing a plurality of sample materials, that are samples of a hydraulic binder, wherein the hydraulic binders contain different compositions of various material components, wherein the process comprises the steps:
a) metering at least one material component into a milling device (20)
b) milling the at least one material component in the milling device (20)
c) metering an activator liquid into the at least one material component
d) homogenizing the at least one material component and the activator liquid to give a sample material,
**characterized in that** the steps a) to d) are repeated to produce a plurality of sample materials, with at least one parameter characteristic for the milling intensity being varied on the milling plant (20),
where the process further comprises the step of determining the reactivity of the plurality of sample materials by means of a calorimetric measuring device (32).

10. The process as claimed in claim 9, wherein the speed of rotation of the milling device and/or the milling time of the at least one material component is varied in step b).

11. The process as claimed in claim 9 or 10, wherein the rheological properties of the plurality of sample materials are determined.

12. The process as claimed in any of claims 9 to 11, wherein the at least one material component is crushed before step b).

13. The process as claimed in any of claims 9 to 12, wherein step a) comprises heating of at least one material component.

14. The process as claimed in any of claims 9 to 13, wherein the particle size of at least part of the milled material components is determined before step d).

## Revendications

1. Appareil (10, 11) pour produire et analyser une pluralité d'échantillons de matériaux, dans lequel les échantillons de matériaux sont des liants hydrauliques et dans lequel les échantillons de matériaux contiennent différentes compositions de divers composants de matériaux, qui comprend
un dispositif de broyage (20) pour broyer les composants matériels,
au moins un premier dispositif de dosage (18 ; 40, 42, 44) pour doser au moins un composant de matériau dans le dispositif de broyage (20),
au moins un deuxième dispositif de dosage (26) pour doser un liquide activateur dans le au moins un composant de matériau broyé,
un dispositif d'homogénéisation (24) pour homogénéiser les composants matériels et le liquide activateur afin d'obtenir un matériau échantillon,
**caractérisé en ce que** l'appareil comporte un dispositif de commande (38) qui est relié au moins au dispositif de broyage (20) et qui est configuré de telle manière qu'il fait varier au moins un paramètre caractéristique de l'intensité de broyage du dispositif de broyage (20) de sorte que, en particulier, la taille des particules des composants du matériau est modifiée, le dispositif de commande (38) étant relié au au moins un premier dispositif de dosage (18 ; 40, 42, 44) et est configuré de telle manière qu'il commande la quantité dosée du composant du matériau et
au moins un dispositif de mesure (32) est prévu pour déterminer la réactivité du matériau échantillon.

2. L'appareil (10 ; 11) selon la revendication 1, dans lequel le dispositif de mesure (32) est un dispositif de mesure calorimétrique.

3. L'appareil (10 ; 11) selon l'une quelconque des revendications précédentes, dans lequel le au moins un premier dispositif de dosage (18 ; 40, 42, 44) est chauffable.

4. Appareil (10 ; 11) selon l'une quelconque des revendications précédentes, dans lequel l'appareil (10 ; 11) comporte un dispositif de broyage (36) qui est situé en amont du dispositif de mouture (20).

5. Appareil (10 ; 11) selon l'une quelconque des revendications précédentes, dans lequel l'appareil (10 ; 11) comporte un dispositif de mesure rhéométrique (34) pour déterminer les propriétés rhéologiques du matériau échantillon.

6. Appareil (10 ; 11) selon l'une quelconque des revendications précédentes, dans lequel l'appareil (10 ; 11) comporte un magasin d'échantillons (30) comportant une pluralité de récipients d'échantillons (28) destinés à recevoir un matériau échantillon.

7. L'appareil (10 ; 11) selon l'une quelconque des revendications précédentes, dans lequel le dispositif de mesure (32) est configuré de telle manière qu'il détermine simultanément la réactivité d'une pluralité de matériaux échantillons.

8. L'appareil (10 ; 11) selon l'une quelconque des revendications précédentes, comprenant au moins un troisième dispositif de dosage (48) pour doser au moins un additif de ciment dans au moins un composant de matériau broyé situé en aval du dispositif de broyage (20).

9. Procédé de production et d'analyse d'une pluralité d'échantillons de matériaux, qui sont des échantillons d'un liant hydraulique, dans lequel les liants hydrauliques contiennent différentes compositions de divers composants de matériaux, dans lequel le procédé comprend les étapes suivantes :
a) doser au moins un composant de matériau dans un dispositif de broyage (20)
b) broyer le au moins un composant de matériau dans le dispositif de broyage (20)
c) doser un liquide activateur dans le au moins un composant de matériau
d) homogénéiser le composant matériel et le liquide activateur pour obtenir un échantillon de matériau,
**caractérisé en ce que** les étapes a) à d) sont répétées pour produire une pluralité d'échantillons de matériaux, au moins un paramètre caractéristique de l'intensité de broyage étant modifié sur l'installation de broyage (20),
le procédé comprenant en outre l'étape consistant à déterminer la réactivité de la pluralité d'échantillons de matériaux à l'aide d'un dispositif de mesure calorimétrique (32).

10. Procédé selon la revendication 9, dans lequel la vitesse de rotation du dispositif de broyage et/ou le temps de broyage du au moins un composant matériel est modifié à l'étape b).

11. Procédé selon la revendication 9 ou 10, dans lequel les propriétés rhéologiques de la pluralité d'échantillons de matériaux sont déterminées.

12. Procédé selon l'une quelconque des revendications 9 à 11, dans lequel le au moins un composant de matériau est broyé avant l'étape b).

13. Procédé selon l'une quelconque des revendications 9 à 12, dans lequel l'étape a) comprend le chauffage d'au moins un composant du matériau.

14. Procédé selon l'une quelconque des revendications 9 à 13, dans lequel la taille des particules d'au moins une partie des composants matériels broyés est déterminée avant l'étape d).
